(19) Europäisches Patentamt
European Patent Office
Office européen des brevets

(11) **EP 4 785 938 A1**

(12) **EUROPEAN PATENT APPLICATION**
published in accordance with Art. 153(4) EPC

(43) Date of publication:
**05.08.2026 Bulletin 2026/32**

(21) Application number: **24872250.6**

(22) Date of filing: **25.09.2024**

(51) International Patent Classification (IPC):
***A61K 31/198*** *(2006.01)* ***A61K 31/385*** *(2006.01)*
***A61K 47/04*** *(2006.01)* ***A61K 47/26*** *(2006.01)*

(52) Cooperative Patent Classification (CPC):
**A61K 31/198; A61K 31/385; A61K 47/02; A61K 47/26**

(86) International application number:
**PCT/JP2024/034101**

(87) International publication number:
**WO 2025/070464 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 JP 2023166659**

(71) Applicant: **Kyowa Pharma Chemical Co., Ltd.**
**Takaoka-shi, Toyama 933-8511 (JP)**

(72) Inventors:
- **TOMONAGA Shoichiro**
**Takaoka-shi, Toyama 933-8511 (JP)**
- **ISHIMARU Hiroaki**
**Takaoka-shi, Toyama 933-8511 (JP)**
- **OHSHIMA Etsuo**
**Tokyo 164-0001 (JP)**

(74) Representative: **Vossius & Partner Patentanwälte Rechtsanwälte mbB**
**Siebertstraße 3**
**81675 München (DE)**

(54) **PHARMACEUTICAL COMPOSITION CONTAINING TRISULFIDE COMPOUND**

(57) Disclosed is a pharmaceutical composition comprising a specific trisulfide compound and a pharmaceutically acceptable carrier, wherein the pharmaceutically acceptable carrier is at least one selected from the group consisting of silica gel-based carriers and saccharides.

***Fig.3***

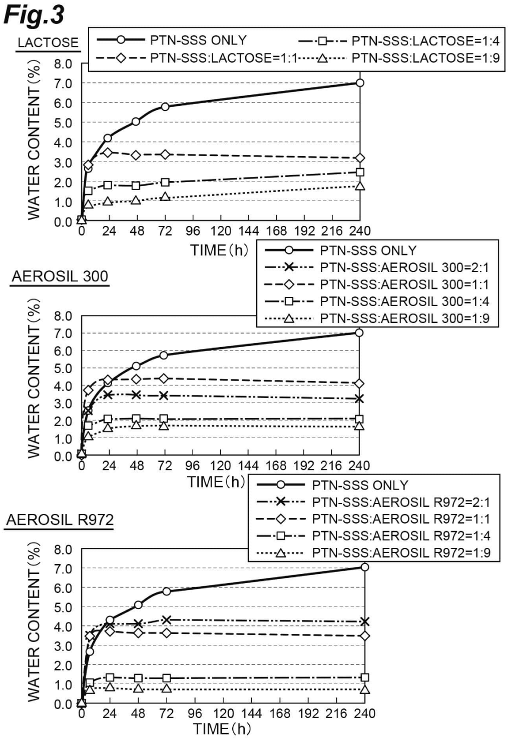


EP 4 785 938 A1

## Description

### Technical Field

The present disclosure relates to a pharmaceutical composition comprising a trisulfide compound.

### Background Art

A compound containing a covalent bond structure formed by three consecutive sulfur atoms is called a trisulfide compound. Since a trisulfide compound has oxidation-reduction ability depending on the possible valence of the constituent sulfur atoms, it is expected to have various physiologically active functions. For example, Non-Patent Literature 1 describes that administration of polysulfide ameliorated neurodegeneration in a Parkinson's disease model mouse.

Patent Literature 1 describes pantethine trisulfide, which is one type of trisulfide compound. Patent Literature 2 describes a trisulfide compound and a clathrate thereof. Patent Literature 3 describes a method for producing glutathione trisulfide, which is one type of trisulfide compound.

### Citation List

### Patent Literature

Patent Literature 1: WO2022/045052
Patent Literature 2: WO2022/045212
Patent Literature 3: WO2021/200487

### Non Patent Literature

Non-Patent Literature 1: Fumiaki Nagashima et al., "Sulfide:quinone oxidoreductase ameliorates neurodegeneration in a murine model of Parkinson's disease", Redox Biology, 59:102562 (2023).

### Summary of Invention

### Technical Problem

In a pharmaceutical composition, the storage stability of an active ingredient in the composition affects the amount of the active ingredient at the time of administration to a subject after the pharmaceutical composition is produced, and thus may affect the therapeutic effect of the pharmaceutical composition. For example, in a case where the hygroscopicity (deliquescence) of the active ingredient is high, in a pharmaceutical composition in which the hygroscopicity is not improved, the active ingredient absorbs moisture and decomposes during the process of storage, and the effective concentration of the active ingredient in the administered subject decreases.

In addition, in a pharmaceutical composition, ease of handling represented by flowability has a significant influence on convenience when a patient takes the composition, or efficiency when subdividing the pharmaceutical composition into doses suitable for administration. In particular, when the flowability of the pharmaceutical composition is low, it becomes difficult to ingest, subdivide, or weigh an accurate amount, which may eventually lead to administration of an unintended dose, or may cause a partial loss of the pharmaceutical composition due to adhesion to equipment.

An object of the present disclosure is to provide a pharmaceutical composition comprising a trisulfide compound.

### Solution to Problem

The present inventors have found that a pharmaceutical composition comprising a trisulfide compound and a specific pharmaceutically acceptable carrier is excellent in storage stability and excellent in handling.

The present disclosure relates to, for example, the following.

[1] A pharmaceutical composition comprising a trisulfide compound and a pharmaceutically acceptable carrier,

wherein the trisulfide compound is at least one selected from the group consisting of:

pantethine trisulfide or a pharmaceutically acceptable salt thereof;
a compound represented by Formula (1):

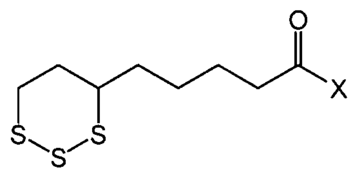

(1)

, wherein X represents $-OR^1$ or $-NR^2R^3$, $R^1$ represents a hydrogen atom or an alkyl group having 1 to 6 carbon atoms, $R^2$ and $R^3$ each independently represent a hydrogen atom or an alkyl group having 1 to 6 carbon atoms, and the alkyl group may have one or more substituents selected from the group consisting of an amino group and a carboxy group, or a pharmaceutically acceptable salt thereof; and
glutathione trisulfide or a pharmaceutically acceptable salt thereof;

and wherein the pharmaceutically acceptable carrier is at least one selected from the group consisting of a silica gel-based carrier and a saccharide.

[2] The pharmaceutical composition according to [1], wherein X in the Formula (1) is -OH.
[3] The pharmaceutical composition according to [1] or [2], wherein the trisulfide compound is pantethine trisulfide or a pharmaceutically acceptable salt thereof.
[4] The pharmaceutical composition according to any one of [1] to [3], wherein the pharmaceutically acceptable carrier is a silica gel-based carrier.
[5] The pharmaceutical composition according to any one of [1] to [4], wherein the trisulfide compound is pantethine trisulfide or a pharmaceutically acceptable salt thereof, and the pharmaceutically acceptable carrier is a silica gel-based carrier.
[6] The pharmaceutical composition according to any one of [1] to [5], wherein the silica gel-based carrier is a hydrophilic silica gel-based carrier.
[7] The pharmaceutical composition according to any one of [1] to [5], wherein the silica gel-based carrier is a hydrophobic silica gel-based carrier.
[8] The pharmaceutical composition according to any one of [1] to [7], wherein a mass ratio of the trisulfide compound to the pharmaceutically acceptable carrier in the pharmaceutical composition is 2:1 to 1:50.
[9] The pharmaceutical composition according to any one of [1] to [8], wherein the pharmaceutical composition is for oral administration or transdermal administration.
[10] The pharmaceutical composition according to any one of [1] to [9], wherein the pharmaceutical composition is a tablet, a capsule, a granule, a powder, an ointment, a cataplasm, or a gel.
[11] The pharmaceutical composition according to any one of [1] to [10], wherein the pharmaceutical composition is a tablet, a capsule, a granule, or a powder.
[12] The pharmaceutical composition according to [1], wherein the pharmaceutically acceptable carrier is a saccharide.
[13] The pharmaceutical composition according to [1] or [12], wherein the pharmaceutically acceptable carrier is lactose.
[14] The pharmaceutical composition according to [1], [12], or [13], wherein the trisulfide compound is pantethine trisulfide or a pharmaceutically acceptable salt thereof, and the pharmaceutically acceptable carrier is lactose.
[15] The pharmaceutical composition according to any one of [1] and [12] to [14], wherein a mass ratio of the trisulfide compound to the pharmaceutically acceptable carrier in the pharmaceutical composition is 1:1 to 1:20.
[16] The pharmaceutical composition according to any one of [1] and [12] to [15], wherein the pharmaceutical composition is for oral administration, pulmonary administration, nasal administration, intraoral administration, rectal administration, or vaginal administration.
[17] The pharmaceutical composition according to any one of [1] and [12] to [16], wherein the pharmaceutical composition is in a form of a tablet, a capsule, a granule, a powder, an inhalation powder, a nasal powder, a sublingual tablet, a troche, a drop, a suppository, or a vaginal suppository.
[18] The pharmaceutical composition according to any one of [1] and [12] to [17], wherein the pharmaceutical composition is a tablet, a capsule, a granule, a powder, an inhalation powder, or a nasal powder.
[19] The pharmaceutical composition according to any one of [1] to [18], which is a carrier-adsorbed composition.
[20] The pharmaceutical composition according to any one of [1] to [19], which is powdery.

[21] The pharmaceutical composition according to any one of [1] to [20], wherein an increase in mass when allowed to stand in air at a temperature of 25 ± 1.5°C and a relative humidity of 43.0 ± 3.5% for 240 hours is 2.0% or less.
[22] The pharmaceutical composition according to any one of [1] to [21], wherein a mass ratio of the trisulfide compound to the pharmaceutically acceptable carrier in the pharmaceutical composition is a mass ratio that achieves an increase in mass of 2.0% or less when allowed to stand in air at a temperature of 25 ± 1.5°C and a relative humidity of 43.0 ± 3.5% for 240 hours.
[23] The pharmaceutical composition according to any one of [1] to [22], wherein an increase in mass when allowed to stand in air at a temperature of 25 ± 1.5°C and a relative humidity of 59.0 ± 5.5% for 240 hours is 6.0% or less.
[24] The pharmaceutical composition according to any one of [1] to [23], wherein a mass ratio of the trisulfide compound to the pharmaceutically acceptable carrier in the pharmaceutical composition is a mass ratio that achieves an increase in mass of 6.0% or less when allowed to stand in air at a temperature of 25 ± 1.5°C and a relative humidity of 59.0 ± 5.5% for 240 hours.
[25] Use or application of a pharmaceutically acceptable carrier in improving storage stability of a trisulfide compound and improving ease of handling in a pharmaceutical composition,

wherein the trisulfide compound is at least one selected from the group consisting of:

pantethine trisulfide or a pharmaceutically acceptable salt thereof;
a compound represented by Formula (1):

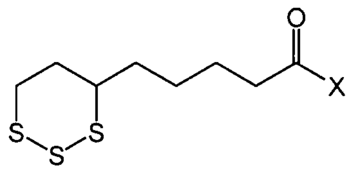

(1)

, wherein X represents $-OR^1$ or $-NR^2R^3$, $R^1$ represents a hydrogen atom or an alkyl group having 1 to 6 carbon atoms, $R^2$ and $R^3$ each independently represent a hydrogen atom or an alkyl group having 1 to 6 carbon atoms, and the alkyl group may have one or more substituents selected from the group consisting of an amino group and a carboxy group, or a pharmaceutically acceptable salt thereof; and
glutathione trisulfide or a pharmaceutically acceptable salt thereof;

and wherein the pharmaceutically acceptable carrier is at least one selected from the group consisting of a silica gel-based carrier and a saccharide.

[26] The use or application according to [25], wherein the use or application is in suppression of moisture absorption and improvement of flowability of the trisulfide compound in the pharmaceutical composition.
[27] A method for improving ease of handling while improving storage stability of a trisulfide compound in a pharmaceutical composition, comprising combining the trisulfide compound and a pharmaceutically acceptable carrier,

wherein the trisulfide compound is at least one selected from the group consisting of:

pantethine trisulfide or a pharmaceutically acceptable salt thereof;
a compound represented by Formula (1):

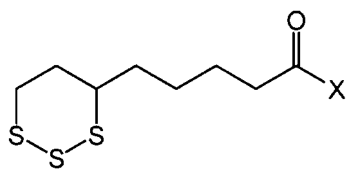

(1)

, wherein X represents $-OR^1$ or $-NR^2R^3$, $R^1$ represents a hydrogen atom or an alkyl group having 1 to 6 carbon atoms, $R^2$ and $R^3$ each independently represent a hydrogen atom or an alkyl group having 1 to 6 carbon

atoms, and the alkyl group may have one or more substituents selected from the group consisting of an amino group and a carboxy group, or a pharmaceutically acceptable salt thereof; and
glutathione trisulfide or a pharmaceutically acceptable salt thereof;

and wherein the pharmaceutically acceptable carrier is at least one selected from the group consisting of a silica gel-based carrier and a saccharide.

[28] The method according to [27], which is a method for improving flowability while suppressing moisture absorption of the trisulfide compound in the pharmaceutical composition.
[29] The use or application according to [25] or [26], or the method according to [27] or [28], wherein the trisulfide compound is pantethine trisulfide or a pharmaceutically acceptable salt thereof.
[30] The use or application according to [25], [26], or [29], or the method according to [27], [28], or [29], wherein the pharmaceutically acceptable carrier is a silica gel-based carrier, and the use, application, or method comprises setting a mass ratio of the trisulfide compound to the pharmaceutically acceptable carrier in the pharmaceutical composition to 2:1 to 1:50.
[31] The use or application according to [25], [26], or [29], or the method according to [27], [28], or [29], wherein the pharmaceutically acceptable carrier is lactose, and the use, application, or method comprises setting a mass ratio of the trisulfide compound to the pharmaceutically acceptable carrier in the pharmaceutical composition to 1:1 to 1:20.
[32] The use or application according to any one of [25], [26], and [29] to [31], or the method according to any one of [27] to [31], wherein the pharmaceutical composition is a carrier-adsorbed composition.
[33] The use or application according to any one of [25], [26], and [29] to [32], or the method according to any one of [27] to [32], wherein the pharmaceutical composition is powdery.
[34] The use or application according to any one of [25], [26], and [29] to [33], or the method according to any one of [27] to [33], comprising ensuring that an increase in mass of the pharmaceutical composition when allowed to stand in air at a temperature of 25 ± 1.5°C and a relative humidity of 43.0 ± 3.5% for 240 hours is 2.0% or less.
[35] The use or application according to any one of [25], [26], and [29] to [34], or the method according to any one of [27] to [34], comprising setting a mass ratio of the trisulfide compound to the pharmaceutically acceptable carrier in the pharmaceutical composition to a mass ratio that achieves an increase in mass of 2.0% or less when allowed to stand in air at a temperature of 25 ± 1.5°C and a relative humidity of 43.0 ± 3.5% for 240 hours.
[36] The use or application according to any one of [25], [26], and [29] to [35], or the method according to any one of [27] to [35], comprising ensuring that an increase in mass of the pharmaceutical composition when allowed to stand in air at a temperature of 25 ± 1.5°C and a relative humidity of 59.0 ± 5.5% for 240 hours is 6.0%.
[37] The use or application according to any one of [25], [26], and [29] to [36], or the method according to any one of [27] to [36], comprising setting a mass ratio of the trisulfide compound to the pharmaceutically acceptable carrier in the pharmaceutical composition to a mass ratio that achieves an increase in mass of 6.0% or less when allowed to stand in air at a temperature of 25 ± 1.5°C and a relative humidity of 59.0 ± 5.5% for 240 hours.

## Advantageous Effects of Invention

According to the present disclosure, a pharmaceutical composition comprising a trisulfide compound is provided. According to the present disclosure, a pharmaceutical composition comprising a trisulfide compound and a pharmaceutically acceptable carrier is provided.
According to the present disclosure, a pharmaceutical composition comprising a trisulfide compound, which is excellent in storage stability and easy to handle, can be provided. According to the present disclosure, a pharmaceutical composition comprising a trisulfide compound, which is excellent in storage stability (e.g., deliquescence) and easy to handle even without coating for enhancing moisture resistance or flowability, can be provided.

## Brief Description of Drawings

[Figure 1] Figure 1 is a diagram showing results of a moisture absorption test of a pharmaceutical composition comprising PTN-SSS, and of PTN-SSS under a condition of a relative humidity of about 20% in Test Example 1.
[Figure 2] Figure 2 is a diagram showing results of a moisture absorption test of a pharmaceutical composition comprising PTN-SSS and a pharmaceutical composition comprising PTN-SSS under a condition of a relative humidity of about 40% in Test Example 1.
[Figure 3] Figure 3 is a diagram showing results of a moisture absorption test of a pharmaceutical composition comprising PTN-SSS and a pharmaceutical composition comprising PTN-SSS under a condition of a relative

humidity of about 60% in Test Example 1.

[Figure 4] Figure 4 is a diagram showing properties of a pharmaceutical composition comprising PTN-SSS and a pharmaceutical composition comprising PTN-SSS after the moisture absorption test under a condition of a relative humidity of about 60% in Test Example 1.

[Figure 5] Figure 5 is a diagram showing stability of a pharmaceutical composition comprising PTN-SSS, a drug substance of PTN-SSS, and an aqueous solution of PTN-SSS under a high temperature condition of 40°C in Test Example 2.

[Figure 6] Figure 6 is a diagram showing a change over time in a value (%) obtained by expressing a peak area derived from PTN-SS generated under the same conditions as in Test Example 2 relative to a peak area derived from PTN-SSS in Test Example 5.

[Figure 7] Figure 7 is a diagram showing a change over time in a value (%) obtained by expressing a peak area derived from PTN-SSSS generated under the same conditions as in Test Example 2 relative to a peak area derived from PTN-SSS in Test Example 5.

## Description of Embodiments

Hereinafter, embodiments for carrying out the present disclosure will be described, but the present disclosure is not limited to the following embodiments.

One embodiment of the present disclosure relates to a pharmaceutical composition comprising a trisulfide compound and a pharmaceutically acceptable carrier. The pharmaceutical composition according to one embodiment of the present disclosure comprises a trisulfide compound as an active ingredient. The pharmaceutical composition according to one embodiment of the present disclosure comprises a therapeutically effective amount of a trisulfide compound.

<Trisulfide Compound>

The trisulfide compound according to one embodiment of the present disclosure is at least one selected from the group consisting of: pantethine trisulfide or a pharmaceutically acceptable salt thereof; a compound represented by Formula (1):

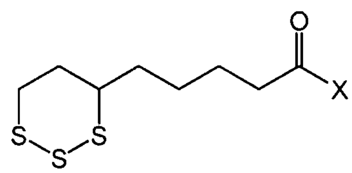

(1)

, wherein X represents $-OR^1$ or $-NR^2R^3$, $R^1$ represents a hydrogen atom or an alkyl group having 1 to 6 carbon atoms, $R^2$ and $R^3$ each independently represent a hydrogen atom or an alkyl group having 1 to 6 carbon atoms, and the alkyl group may have one or more substituents selected from the group consisting of an amino group and a carboxy group (hereinafter also described as "Compound (1)"), or a pharmaceutically acceptable salt thereof; and glutathione trisulfide or a pharmaceutically acceptable salt thereof. These trisulfide compounds may be comprised in the pharmaceutical composition in a form other than a free form or a pharmaceutically acceptable salt thereof, and for example, may be comprised in a form of a solvate thereof (e.g., a hydrate).

In the present disclosure, examples of the pharmaceutically acceptable salt include salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; and salts with amino acids such as arginine.

Pantethine trisulfide is represented by the following Formula (2A). Pantethine trisulfide can be prepared, for example, according to the method described in Patent Literature 1.

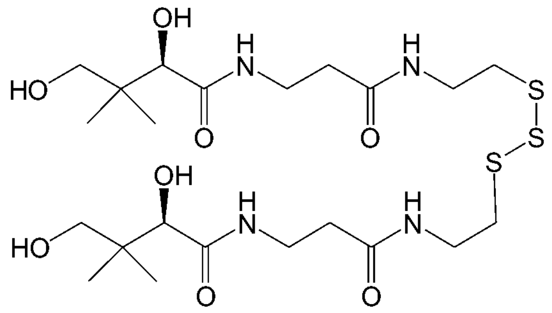

(2A)

Glutathione trisulfide is represented by the following Formula (2). Glutathione trisulfide can be prepared, for example, according to the method described in Patent Literature 3.

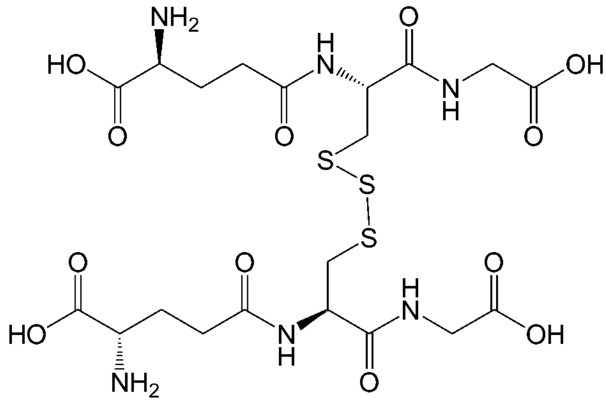

(2)

The pharmaceutically acceptable salt of glutathione trisulfide and pantethine trisulfide is preferably an amino acid salt or an alkali metal salt, and more preferably an arginine salt or a sodium salt. The pharmaceutically acceptable salt of Compound (1) is preferably a salt with an alkali metal, and more preferably a sodium salt.

Compound (1) can be prepared, for example, according to the method described in Patent Literature 2.

In one embodiment, Compound (1) is a compound represented by Formula (3):

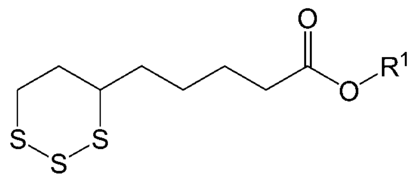

(3)

, wherein $R^1$ represents a hydrogen atom or an alkyl group having 1 to 6 carbon atoms, and $R^1$ may be, for example, a hydrogen atom, a methyl group, an ethyl group, a propyl group, a butyl group, a pentyl group, or a hexyl group. Preferably, $R^1$ is a hydrogen atom, and in this case, the compound represented by Formula (3) is lipoic acid trisulfide represented by Formula (4).

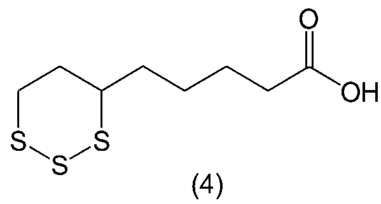

(4)

In another embodiment, Compound (1) is a compound represented by Formula (5):

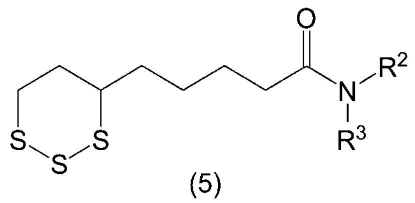

(5)

, wherein $R^2$ and $R^3$ each independently represent a hydrogen atom or an alkyl group having 1 to 6 carbon atoms, and the alkyl group may have one or more substituents selected from the group consisting of an amino group and a carboxy group. $R^1$ and $R^2$ may be an alkyl group such as a hydrogen atom, a methyl group, an ethyl group, a propyl group, a butyl group, a pentyl group, or a hexyl group. These alkyl groups may have one or both of an amino group and a carboxy group as substituents. $R^1$ and $R^2$ may be, for example, a group represented by Formula (6) (wherein * represents a bond). Specific examples of the compound represented by Formula (5) include a compound in which $R^2$ and $R^3$ are both hydrogen atoms, and a compound in which $R^2$ is a hydrogen atom and $R^3$ is a group represented by Formula (6).

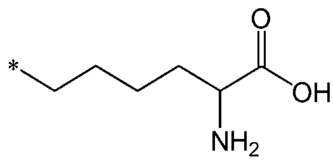

(6)

In a preferred embodiment, the trisulfide compound according to the present disclosure may be pantethine trisulfide or a pharmaceutically acceptable salt thereof, Compound (1) or a pharmaceutically acceptable salt thereof, or glutathione trisulfide or a pharmaceutically acceptable salt thereof; in a more preferred embodiment, it may be pantethine trisulfide or a pharmaceutically acceptable salt thereof, or glutathione trisulfide or a pharmaceutically acceptable salt thereof; and in a particularly preferred embodiment, it may be pantethine trisulfide or a pharmaceutically acceptable salt thereof.

The pharmaceutically acceptable carrier according to one embodiment of the present disclosure is at least one selected from the group consisting of a silica gel-based carrier and a saccharide.

The silica gel-based carrier according to the pharmaceutical composition of the present disclosure may be any carrier that can be used as a pharmaceutically acceptable carrier. The silica gel-based carrier according to the present disclosure may be, for example, fumed silica or colloidal silica, and in a preferred embodiment, it may be fumed silica.

The silica gel-based carrier according to the present disclosure may be, for example, a hydrophobic (i.e., surface-treated) or hydrophilic (i.e., non-surface-treated) silica gel-based carrier. The surface treatment when the silica gel-based carrier according to the present disclosure is hydrophobic is not particularly limited as long as it is usually performed by those skilled in the art, but may be, for example, dimethylsilyl modification, trimethylsilyl modification, octylsilyl modification, or dimethylpolysiloxane modification, and in a preferred embodiment, it may be dimethylsilyl modification, and the dimethylsilyl modification may be performed by surface treatment using dimethyldichlorosilane.

The specific surface area determined by the BET method (method of Brunauer, Emmett and Teller) of the fumed silica which is the silica gel-based carrier according to the pharmaceutical composition of the present disclosure may be, for example, 30 to 1000 $m^2$/g, 45 to 660 $m^2$/g, 60 to 500 $m^2$/g, or 90 to 330 $m^2$/g, and may also be, for example, 30 to 400 $m^2$/g, 45 to 260 $m^2$/g, 60 to 200 $m^2$/g, or 90 to 130 $m^2$/g.

The pH in a 4% aqueous dispersion of the fumed silica which is the silica gel-based carrier according to the pharmaceutical composition of the present disclosure may be, for example, 3.0 to 7.0, 3.3 to 6.5, 3.6 to 6.0, or 3.8 to 5.5.

The bulk density of the fumed silica which is the silica gel-based carrier according to the pharmaceutical composition of the present disclosure may be, for example, 10 to 250 g/L, 20 to 125 g/L, 25 to 100 g/L, or 40 to 60 g/L.

Examples of the silica gel-based carrier according to the pharmaceutical composition of the present disclosure include AEROSIL (registered trademark) series (EVONIK), SYLOID (registered trademark) series (GRACE), and CAB-O-SIL (registered trademark) series (CABOT), and in a preferred embodiment, it may be AEROSIL (registered trademark) series, in a more preferred embodiment, it may be AEROSIL (registered trademark) R972, AEROSIL (registered trademark) 300, or AEROSIL (registered trademark) 200, and in a particularly preferred embodiment, it may be AEROSIL (registered trademark) R972. AEROSIL (registered trademark) R972 is hydrophobic fumed silica surface-treated with dimethyldichlorosilane, having a specific surface area determined by the BET method of 90 to 130 $m^2$/g, a pH in a 4% aqueous dispersion of 4.0 to 5.5, and an apparent bulk density of about 50 g/L. AEROSIL (registered trademark) 300 is

hydrophilic fumed silica, having a specific surface area determined by the BET method of 270 to 330 $m^2/g$, a pH in a 4% aqueous dispersion of 3.8 to 4.3, and an apparent bulk density of about 50 g/L.

The saccharide according to the pharmaceutical composition of the present disclosure is not particularly limited as long as it is a saccharide that can be used as a pharmaceutically acceptable carrier, and may be, for example, a monosaccharide, an oligosaccharide, or a polysaccharide, in a preferred embodiment, it may be a monosaccharide or an oligosaccharide, and in a more preferred embodiment, it may be a monosaccharide or a disaccharide. In the present disclosure, the oligosaccharide means a saccharide composed of glycosidic bonds of 2 or more and 6 or less monosaccharides (disaccharide to hexasaccharide) linked by glycosidic bond(s), and in the present disclosure, the polysaccharide means a saccharide composed of glycosidic bonds of 7 or more monosaccharides linked by glycosidic bonds.

A molecular weight of the saccharide according to the pharmaceutical composition of the present disclosure may be, for example, 10000 or less, 3000 or less, 1000 or less, 500 or less, or 350 or less.

Examples of the monosaccharide according to the pharmaceutical composition of the present disclosure include glucose (grape sugar), fructose (fruit sugar), xylitol, D-sorbitol, and D-mannitol. Examples of the oligosaccharide according to the pharmaceutical composition of the present disclosure include lactose (milk sugar), sucrose (cane sugar), maltose (malt sugar), trehalose, and cyclodextrin. Examples of the polysaccharide according to the pharmaceutical composition of the present disclosure include starch, hypromellose, and dextran. In one embodiment of the present disclosure, the saccharide according to the pharmaceutical composition may be a saccharide other than cyclodextrin.

The saccharide according to the pharmaceutical composition of the present disclosure may be, for example, lactose (milk sugar), glucose (grape sugar), sucrose (cane sugar), maltose (malt sugar), or trehalose, in a preferred embodiment, it may be lactose or sucrose, and in a more preferred embodiment, it may be lactose.

In the pharmaceutical composition according to one embodiment of the present disclosure, the trisulfide compound may be pantethine trisulfide or a pharmaceutically acceptable salt thereof, and the pharmaceutically acceptable carrier may be a silica gel-based carrier. In the pharmaceutical composition according to one embodiment of the present disclosure, the trisulfide compound may be pantethine trisulfide or a pharmaceutically acceptable salt thereof, and the pharmaceutically acceptable carrier may be a saccharide.

In the pharmaceutical composition according to one embodiment of the present disclosure, the mass ratio of the trisulfide compound to the pharmaceutically acceptable carrier may be 2:1 to 1:50, 2:1 to 1:20, 2:1 to 1:9, 1.8:1 to 1:6, 1.6:1 to 1:4, 1.4:1 to 1:2, or 1.2:1 to 1:1.2. When the mass ratio of the trisulfide compound to the pharmaceutically acceptable carrier is within the above range, moisture absorption in the pharmaceutical composition is suppressed, and handling becomes easier. The mass of the trisulfide compound in the above mass ratio may be a mass converted to a free form.

In the pharmaceutical composition according to one embodiment of the present disclosure, when the pharmaceutically acceptable carrier is a silica gel-based carrier, the mass ratio of the trisulfide compound to the silica gel-based carrier may be 2:1 to 1:50, 2:1 to 1:20, 2:1 to 1:9, 1.8:1 to 1:6, 1.6:1 to 1:4, 1.4:1 to 1:2, or 1.2:1 to 1:1.2. When the mass ratio of the trisulfide compound to the pharmaceutically acceptable carrier is within the above range, moisture absorption in the pharmaceutical composition is suppressed, and handling becomes easier. The mass of the trisulfide compound in the above mass ratio may be a mass converted to a free form.

In the pharmaceutical composition according to one embodiment of the present disclosure, when the pharmaceutically acceptable carrier is a saccharide, the mass ratio of the trisulfide compound to the saccharide may be 1:1 to 1:20, 1:1 to 1:14, 1:1 to 1:9, 1:1 to 1:6, 1:1 to 1:4, 1:1 to 1:2, or 1:1 to 1:1.2. When the mass ratio of the trisulfide compound to the pharmaceutically acceptable carrier is within the above range, moisture absorption in the pharmaceutical composition is remarkably suppressed. The mass of the trisulfide compound in the above mass ratio may be a mass converted to a free form.

The pharmaceutical composition according to one embodiment of the present disclosure may further comprise an additive as another component in addition to the trisulfide compound and the pharmaceutically acceptable carrier. Examples of the additive include stabilizers such as amino acids (L-arginine, etc.), water-soluble polymers (HES (hydroxyethyl starch), PVP (polyvinylpyrrolidone), etc.), and nonionic surfactants (polysorbate, poloxamer, etc.), isotonizing agents such as sodium chloride, and excipients such as glycine and sodium chloride.

In the administration of the pharmaceutical composition according to one embodiment of the present disclosure, the dose of the trisulfide compound is preferably 0.5 to 800 mg per 1 kg of body weight per day (0.5 to 800 mg/kg/day), more preferably 1 to 400 mg/kg/day, and still more preferably 2 to 200 mg/kg/day.

The frequency of administration of the pharmaceutical composition according to one embodiment of the present disclosure can be 1 to 8 times a day or 1 to 4 times a day. With such a frequency of administration, it is considered that the burden of taking medication on the patient is reduced and medication compliance is improved.

The pharmaceutical composition according to one embodiment of the present disclosure may be, for example, for oral administration, transdermal administration, pulmonary administration, ophthalmic administration, nasal administration, intraoral administration, rectal administration, or vaginal administration. In other words, the pharmaceutical composition according to one embodiment of the present disclosure may be in a form suitable for oral administration, transdermal administration, pulmonary administration, ophthalmic administration, nasal administration, intraoral administration, rectal administration, or vaginal administration. In the pharmaceutical composition according to one embodiment of the present disclosure, when the pharmaceutically acceptable carrier is a silica gel-based carrier, the pharmaceutical composition may be, for example, for oral administration or transdermal administration, and in a preferred embodiment, it may be for oral administration. In the pharmaceutical composition according to one embodiment of the present disclosure, when the pharmaceutically acceptable carrier is a saccharide, the pharmaceutical composition may be, for example, for oral administration, pulmonary administration, ophthalmic administration, nasal administration, intraoral administration, rectal administration, or vaginal administration, in a preferred embodiment, it may be for oral administration, pulmonary administration, nasal administration, or intraoral administration, and in a more preferred embodiment, it may be for oral administration.

The pharmaceutical composition according to one embodiment of the present disclosure may be, for example, in a form of a tablet, a capsule, a granule, a powder, an ointment, a cataplasm, a gel, an inhalation powder, a nasal powder, a sublingual tablet, a troche, a drop, a suppository, or a vaginal suppository. In the pharmaceutical composition according to one embodiment of the present disclosure, when the pharmaceutically acceptable carrier is a silica gel-based carrier, the pharmaceutical composition may be, for example, in a form of a tablet, a capsule, a granule, a powder, an ointment, a cataplasm, or a gel, and in a preferred embodiment, it may be a tablet, a capsule, a granule, or a powder. In the pharmaceutical composition according to one embodiment of the present disclosure, when the pharmaceutically acceptable carrier is a saccharide, the pharmaceutical composition may be, for example, in a form of a tablet, a capsule, a granule, a powder, an inhalation powder, a nasal powder, a sublingual tablet, a troche, a drop, a suppository, or a vaginal suppository, in a preferred embodiment, it may be in a form of a tablet, a capsule, a granule, a powder, an inhalation powder, or a nasal powder, and in a more preferred embodiment, it may be in a form of a tablet, a capsule, a granule, or a powder. In these cases, the tablet, capsule, granule, or powder may be for oral administration, the ointment or cataplasm may be for transdermal administration, the gel may be for ophthalmic administration, nasal administration, intraoral administration, or vaginal administration, the inhalation powder may be for pulmonary administration, the nasal powder may be for nasal administration, the sublingual tablet, troche, or drop may be for intraoral administration, the suppository may be for rectal administration, and the vaginal suppository may be for vaginal administration.

The pharmaceutical composition according to one embodiment of the present disclosure may be a carrier-adsorbed composition. That is, the pharmaceutical composition according to one embodiment of the present disclosure may be a composition in which the trisulfide compound and an additive as another component are adsorbed to the pharmaceutically acceptable carrier. In the present disclosure, the carrier-adsorbed composition is distinguished from a solid dispersion using a polymer. The pharmaceutical composition according to one embodiment of the present disclosure may be in powder form.

In the pharmaceutical composition according to one embodiment of the present disclosure, hygroscopicity is suppressed. In the present disclosure, that hygroscopicity is suppressed may mean, for example, that an increase in mass when allowed to stand in air at a temperature of 25 ± 1.5°C and a relative humidity of 43.0 ± 3.5% for 240 hours is 2.0% or less, 1.5% or less, or 1.0% or less, or that the mass ratio of the trisulfide compound to the pharmaceutically acceptable carrier in the pharmaceutical composition is a mass ratio that achieves the above mass increase. In the present disclosure, that hygroscopicity is suppressed may mean, for example, that an increase in mass when allowed to stand in air at a temperature of 25 ± 1.5°C and a relative humidity of 43.0 ± 3.5% for 240 hours is 90% or less, 80% or less, 70% or less, 60% or less, 50% or less, 40% or less, 30% or less, or 20% or less of an increase in mass when a drug substance of the trisulfide compound comprised in the pharmaceutical composition is allowed to stand under similar conditions, or that the mass ratio of the trisulfide compound to the pharmaceutically acceptable carrier in the pharmaceutical composition is a mass ratio that achieves the above mass increase. In the present disclosure, that hygroscopicity is suppressed may mean, for example, that an increase in mass when allowed to stand in air at a temperature of 25 ± 1.5°C and a relative humidity of 59.0 ± 5.5% for 240 hours is 6.0% or less, 5.0% or less, 4.5% or less, 4.0% or less, 3.0% or less, 2.0% or less, or 1.0% or less, or that the mass ratio of the trisulfide compound to the pharmaceutically acceptable carrier in the pharmaceutical composition is a mass ratio that achieves the above mass increase. In the present disclosure, that hygroscopicity is suppressed may mean, for example, that an increase in mass when allowed to stand in air at a temperature of 25 ± 1.5°C and a relative humidity of 59.0 ± 5.5% for 240 hours is 90% or less, 80% or less, 70% or less, 60% or less, 50% or less, 40% or less, 30% or less, or 20% or less of an increase in mass when a drug substance of the trisulfide compound comprised in the pharmaceutical composition is allowed to stand under similar conditions, or that the mass ratio of the trisulfide compound to the pharmaceutically acceptable carrier in the pharmaceutical composition is a mass ratio that achieves the above mass increase. Then, by further subjecting the pharmaceutical composition in which hygroscopicity is suppressed by comprising a carrier as described above to formulation for the purpose of moisture protection such as a capsule, a formulation having further improved moisture resistance can be obtained.

The pharmaceutical composition according to one embodiment of the present disclosure is excellent in storage stability. In the present disclosure, that the pharmaceutical composition is excellent in storage stability may mean, for example, that the above-mentioned moisture absorption is suppressed in the pharmaceutical composition. In addition, in the present disclosure, that the pharmaceutical composition is excellent in storage stability may mean, for example, that a

ratio of decomposition of the trisulfide compound when allowed to stand in air at 40°C under a light-shielded condition for 14 days is 18% or less, 15% or less, 10% or less, 5% or less, 4% or less, 3% or less, or 2% or less.

The pharmaceutical composition according to one embodiment of the present disclosure is excellent in flowability. In the present disclosure, that the pharmaceutical composition is excellent in flowability may mean, for example, that an angle of repose (static angle of repose) is 45° or less, 40° or less, 35° or less, 30° or less, or 25° or less. In addition, in the present disclosure, that the pharmaceutical composition is excellent in flowability may mean, for example, that compressibility is 25% or less, 20% or less, 15% or less, 13% or less, 10% or less, or 9% or less. The angle of repose may be a value measured according to the method described in, for example, "The Japanese Pharmacopoeia, 18th Edition, General Information, Powder Flow <G2-3-171>".

The pharmaceutical composition according to one embodiment of the present disclosure is excellent in ease of handling (easy to handle). In the present disclosure, that the pharmaceutical composition is easy to handle may mean, for example, that the pharmaceutical composition is excellent in the above-mentioned flowability. In addition, examples of the pharmaceutical composition being easy to handle include suppression of excessive increase in viscosity and of formation of lumps, low adhesion to a medicine spoon, subdividing equipment, etc., and low scattering due to wind, etc.

Another aspect of the present disclosure is use or application of a pharmaceutically acceptable carrier in improving storage stability of a trisulfide compound and improving ease of handling in a pharmaceutical composition. In one embodiment, the use or application may be use or application in suppression of moisture absorption and improvement of flowability of a trisulfide compound in a pharmaceutical composition. As the trisulfide compound, the pharmaceutically acceptable carrier, and the pharmaceutical composition in these cases, those described above as the pharmaceutical composition according to one embodiment of the present disclosure can be used.

Another aspect of the present disclosure is a method for improving ease of handling while improving storage stability of a trisulfide compound in a pharmaceutical composition, comprising combining the trisulfide compound and a pharmaceutically acceptable carrier. In one embodiment, the method may be a method for improving flowability while suppressing moisture absorption of a trisulfide compound in a pharmaceutical composition. In one embodiment, the method may be a method for improving ease of handling while improving storage stability of a trisulfide compound in a pharmaceutical composition comprising the trisulfide compound, comprising adding at least one carrier selected from the group consisting of a silica gel-based carrier and a saccharide to the pharmaceutical composition. As the trisulfide compound, the pharmaceutically acceptable carrier, and the pharmaceutical composition in these cases, those described above as the pharmaceutical composition according to one embodiment of the present disclosure can be used.

In one embodiment of these uses, applications, or methods, the pharmaceutically acceptable carrier is a silica gel-based carrier, and the use, application, or method comprises setting the mass ratio of the trisulfide compound to the pharmaceutically acceptable carrier in the pharmaceutical composition to 2:1 to 1:50, 2:1 to 1:20, 2:1 to 1:9, 1.8:1 to 1:6, 1.6:1 to 1:4, 1.4:1 to 1:2, or 1.2:1 to 1:1.2. When the mass ratio of the trisulfide compound to the pharmaceutically acceptable carrier is within the above range, moisture absorption in the pharmaceutical composition is suppressed, and handling becomes easier. The mass of the trisulfide compound in the above mass ratio may be a mass converted to a free form.

In one embodiment of these uses, applications, or methods, the pharmaceutically acceptable carrier is lactose, and the use, application, or method comprises setting the mass ratio of the trisulfide compound to the pharmaceutically acceptable carrier in the pharmaceutical composition to 1:1 to 1:20, 1:1 to 1:14, 1:1 to 1:9, 1:1 to 1:6, 1:1 to 1:4, 1:1 to 1:2, or 1:1 to 1:1.2. When the mass ratio of the trisulfide compound to the pharmaceutically acceptable carrier is within the above range, moisture absorption in the pharmaceutical composition is remarkably suppressed. The mass of the trisulfide compound in the above mass ratio may be a mass converted to a free form.

## Examples

Hereinafter, the present disclosure will be described more specifically using Examples, but the present disclosure is not limited to the following Examples.

In the following Examples, pantethine trisulfide is also referred to as PTN-SSS. In the following Examples, pantethine is also referred to as PTN-SS. PTN-SS is a compound represented by the following structural formula.

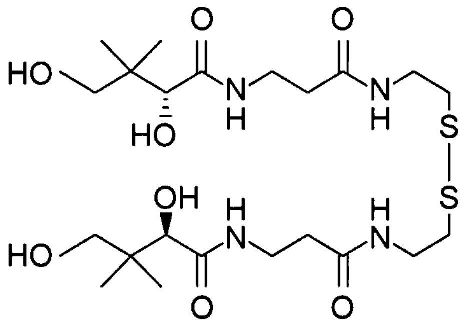

In addition, in the following Examples, pantethine tetrasulfide is also referred to as PTN-SSSS. PTN-SSSS is a compound in which one additional sulfur atom is linearly inserted into the trisulfide moiety of PTN-SSS, and is represented by the following structural formula.

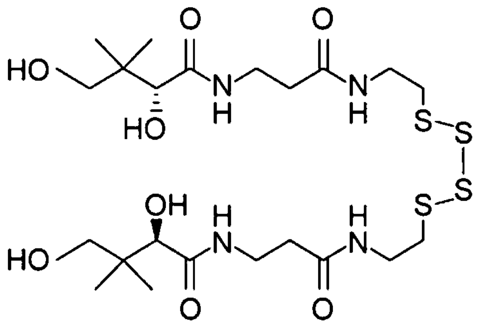

PTN-SSS used in the following Examples was prepared according to the method described in Patent Literature 1.

<Preparation Example 1: Preparation of Composition Comprising PTN-SSS and Silica Gel-Based Carrier>

After dissolving 1 g of PTN-SSS in 10 mL of methanol, AEROSIL 300 or AEROSIL R972 was added to the solution so that the mass ratio of PTN-SSS to AEROSIL 300 or AEROSIL R972 was 2:1, 1:1, 1:4, or 1:9, and the mixture was stirred for 30 minutes. Thereafter, methanol was distilled off under reduced pressure, and the residue was dried under reduced pressure.

<Preparation Example 2: Preparation of Composition Comprising PTN-SSS and Saccharide>

After dissolving 1 g of PTN-SSS in 10 mL of methanol, lactose was added to the solution so that the mass ratio of PTN-SSS to lactose was 2:1, 1:1, 1:4, or 1:9, and the mixture was stirred for 30 minutes. Thereafter, methanol was distilled off under reduced pressure, and the residue was dried under reduced pressure.

<Test Example 1: Moisture Absorption Test of Composition Comprising PTN-SSS>

For PTN-SSS and the compositions prepared in Preparation Examples 1 and 2, storage stability (hygroscopicity, deliquescence) was evaluated from mass change under conditions of relative humidity of about 20%, 40%, and 60%. As the evaluation of hygroscopicity, the samples prepared in Preparation Examples 1 and 2 were stored in a desiccator in which humidity was kept constant by a saturated salt method, the weight was measured from the start of storage to 72 hours, and the amount of moisture absorption was calculated from the weight change.

The results of mass change under the condition of relative humidity of about 20% (25.0 $\pm$ 2.5%, humidity adjusted by saturated salt method of potassium acetate saturated aqueous solution) are shown in Figure 1. The results of mass change under the condition of relative humidity of about 40% (43.0 $\pm$ 3.5%, humidity adjusted by saturated salt method of potassium carbonate saturated aqueous solution) are shown in Figure 2. The results of mass change under the condition of relative humidity of about 60% (59.0 $\pm$ 5.5%, humidity adjusted by saturated salt method of potassium iodide saturated aqueous solution) are shown in Figure 3. In addition, photographs of the respective compositions 72 hours after the start of the test under the condition of relative humidity of about 60% are shown in Figure 4. Note that Figure 4 also shows photographs before the start of the test and at the time points of 24 hours and 72 hours after the start of the test under the condition of relative humidity of 60% as controls. According to Figures 1 to 3, in PTN-SSS as a control, an increase in mass

occurred under the conditions of relative humidity of about 40% and about 60%, and moisture absorption continued even after 10 days. In addition, even under the condition of relative humidity of about 20%, deliquescence of PTN-SSS was confirmed, and the solid state could not be maintained. On the other hand, in the composition comprising a saccharide (lactose) at a mass ratio of 1:1 or more with respect to PTN-SSS, and the composition comprising a silica gel-based carrier (AEROSIL 300, AEROSIL R972) at a mass ratio of 2:1 or more with respect to PTN-SSS, mass increase as much as PTN-SSS did not occur even under the conditions of relative humidity of about 40% and about 60%, moisture absorption was suppressed, and the powder state could be maintained for 10 days. Specifically, under the condition of relative humidity of about 40%, the mass increase of the composition after 10 days was suppressed to 2.0% or less, and under the condition of relative humidity of about 60%, the mass increase of the composition after 10 days was suppressed to 6.0% or less, 5.0% or less, and 4.5% or less. From these results, it was shown that the composition comprising the trisulfide compound and the saccharide or silica gel-based carrier as a pharmaceutically acceptable carrier has suppressed hygroscopicity and is excellent in storage stability.

<Test Example 2: Stability Test of Composition Comprising PTN-SSS under High Temperature Condition>

For PTN-SSS (drug substance), an aqueous solution of PTN-SSS, a composition comprising PTN-SSS and silica gel, and the compositions prepared in Preparation Examples 1 and 2, stability under a high temperature condition was tested. Note that the aqueous solution of PTN-SSS is a group simulating a case where the drug substance of PTN-SSS absorbed moisture, and PTN-SSS was set at a high concentration (80%) condition.

As the aqueous solution of PTN-SSS used in Test Example 2, 80% PTN-SSS was prepared by weighing 245 mg of PTN-SSS and adding 61 μL of purified water. In addition, 50% PTN-SSS (silica gel) was prepared by dissolving 1 g of PTN-SSS in 10 mL of methanol, adding the solution to silica gel so that the mass ratio of PTN-SSS to silica gel (Kanto Chemical, Silica Gel 60N, spherical, neutral) was 1:1, stirring the mixture for 30 minutes, and then distilling off methanol under reduced pressure and drying under reduced pressure.

Each sample was weighed into a brown 20 mL screw vial and stored in a thermostat at 40°C. Sampling was performed at appropriate time points from the start of the stability test, and HPLC purity was measured by HPLC for up to 14 days. The analysis conditions of HPLC were as shown below.
Detector: Ultraviolet absorptiometer (measurement wavelength: 220 nm)
Column: LiChrosorb RP-18 (Kanto Chemical, 4.0 x 250 mm, 5 μm)
Column temperature: Constant temperature around 40°C
Mobile phase A: Phosphoric acid aqueous solution (pH 3)
Mobile phase B: Methanol
Delivery of mobile phase: The concentration gradient was controlled by changing the mixing ratio of mobile phase A and mobile phase B as shown in Table 1.

[Table 1]

| Time after injection (min) | Mobile phase A (vol%) | Mobile phase B (vol%) |
|---|---|---|
| 0 to 5 | 100 | 0 |
| 5 to 15 | 100 -> 50 | 0 -> 50 |
| 15 to 20 | 50 | 50 |
| 20 to 21 | 50 -> 100 | 50 -> 0 |
| 21 to 35 | 100 | 0 |

Flow rate: 0.6 mL/min
Injection volume: 10 μL

The results are shown in Figure 5. In Figure 5, 50% (lactose) indicates a composition in which the mass ratio of PTN-SSS to lactose is 1:1, 50% (AEROSIL) indicates a composition in which the mass ratio of PTN-SSS to AEROSIL 300 is 1:1, 20% (lactose) indicates a composition in which the mass ratio of PTN-SSS to lactose is 1:4, and 20% (AEROSIL) indicates a composition in which the mass ratio of PTN-SSS to AEROSIL 300 is 1:4. According to Figure 5, in the aqueous solution of PTN-SSS, the content of PTN-SSS after 14 days under the condition of 40°C decreased to 85% or less, whereas in PTN-SSS and the composition comprising a saccharide or a silica gel-based carrier as a pharmaceutically acceptable carrier, no remarkable decrease in the content of PTN-SSS was observed. From this, it was revealed that the composition comprising the trisulfide compound and the saccharide or silica gel-based carrier as a pharmaceutically acceptable carrier is stable against high temperature conditions and is excellent in storage stability.

<Test Example 3: Ease of Handling of Composition Comprising PTN-SSS>

For the composition after 72 hours whose photograph is shown in Figure 4 obtained in Test Example 1, and the composition after standing for 72 hours under the condition of relative humidity of about 20% or about 40% in the same manner as Figure 4, the properties and handling of the powder were visually confirmed.
The results under the condition of relative humidity of about 60% (60% RH) are shown in Table 2. The results under the conditions of relative humidity of about 40% (40% RH) and about 20% (20% RH) are shown in Table 3. In Table 2 and Table 3, A indicates that handling was extremely good, B indicates that handling was good, and C indicates that handling was not very good. In addition, in Table 2 and Table 3, 1) indicates that it did not turn into powder and had high viscosity, 2) indicates that it was easy to handle but formed lumps when absorbing moisture and adhered to a medicine spoon etc., and 3) indicates that the amount of carrier was large and it was slightly difficult to handle because it was scattered by a slight breeze etc. According to the results of Table 2 and Table 3, it was revealed that the composition comprising a saccharide at a mass ratio of 1:1 to 1:9 with respect to PTN-SSS and the composition comprising a silica gel-based carrier at a mass ratio of 2:1 to 1:9 with respect to PTN-SSS are compositions superior in ease of handling.

[Table 2]

| Properties and Handling (60% RH) | | | | |
|---|---|---|---|---|
| | 2:1 | 1:1 | 1:4 | 1:9 |
| Lactose | C1) | B2) | B2) | B2) |
| AEROSIL 300 | B2) | A | B3) | B3) |
| AEROSIL R972 | B2) | A | A | A |

[Table 3]

| Properties and Handling (20, 40% RH) | | | | |
|---|---|---|---|---|
| | 2:1 | 1:1 | 1:4 | 1:9 |
| Lactose | C1) | A | A | A |
| AEROSIL 300 | A | A | B3) | B3) |
| AEROSIL R972 | A | A | A | A |

<Test Example 4: Flowability of Composition Comprising PTN-SSS>

For the composition comprising PTN-SSS and silica gel, and the compositions prepared in Preparation Examples 1 and 2, the flowability thereof was evaluated.
Based on the measurement method described in "The Japanese Pharmacopoeia, 18th Edition, General Information, Powder Flow <G2-3-171>", the angle of repose and compressibility were measured according to the protocol described below.

Measurement of Angle of Repose

1. A funnel was set at a height of about 2 cm from the bottom surface where the powder was dropped.
2. The powder was dropped, and the height of the formed pile of powder was measured.
3. The length of the bottom surface was measured.
4. The angle of repose was calculated by the following formula.

$$\tan(\alpha) = \frac{\text{Height of pile}}{\text{Length of bottom surface}/2}$$

Measurement of Compressibility

1. The powder was gently put into a 10 mL graduated cylinder.
2. The loose bulk density ($V_0$) was recorded.
3. The graduated cylinder was tapped until the bulk no longer change.
4. The tapped bulk density ($V_f$) was recorded.
5. The compressibility was calculated by the following formula.

$$\text{Compressibility} = \frac{V_0 - V_f}{V_0} \times 100$$

The results of measuring the angle of repose and compressibility of the composition in which the mass ratio of PTN-SSS to the carrier (lactose, AEROSIL 300, AEROSIL R972) is 1:1 to 1:9, and the carrier alone are shown in Table 4 and Table 5, respectively. A scale of general flowability for the angle of repose and compressibility, which was used as a criterion for evaluating whether the flowability is good in Table 4 and Table 5, is shown in Table 6. In addition, in Table 4 and Table 5, 1) indicates that blocking occurred and measurement was impossible. In addition, the angle of repose in the composition in which the mass ratio of PTN-SSS to silica gel was 1:4 was 23°, and the compressibility was 8.5. From these results, it was revealed that a composition comprising a saccharide at a mass ratio of 1:4 to 1:9 with respect to PTN-SSS and a composition comprising a silica gel-based carrier at a mass ratio of 1:1 to 1:9 with respect to PTN-SSS are compositions excellent in flowability.

[Table 4]

| Angle of Repose of Composition with Each Carrier | | | | |
|---|---|---|---|---|
| | 1:1 | 1:4 | 1:9 | Carrier only |
| Lactose | -1) | Good | Good | Poor |
| | | 34° | 35° | 61° |
| AEROSIL 300 | Extremely good 25° | Extremely good 24° | Extremely good 22° | Extremely good 23° |
| AEROSIL R972 | Extremely good 25° | Extremely good 23° | Extremely good 22° | Extremely good 28° |

[Table 5]

| Compressibility of Composition with Each Carrier | | | | |
|---|---|---|---|---|
| | 1:1 | 1:4 | 1;9 | Carrier only |
| Lactose | _1) | Good 12% | Good 13% | Slightly poor 28% |
| AEROSIL 300 | Extremely good 10% | Extremely good 7% | Slightly good 19% | Fair 21% |
| AEROSIL R972 | Extremely good 6% | Extremely good 4% | Slightly good 18% | Fair 21% |

[Table 6]

| Degree of Flowability | Angle of Repose (◦) | Compressibility (%) |
|---|---|---|
| Extremely good | 25 to 30 | ≤ 10 |
| Good | 31 to 35 | 11 to 15 |
| Slightly good | 36 to 40 | 16 to 20 |
| Fair | 41 to 45 | 21 to 25 |
| Slightly poor | 46 to 55 | 26 to 31 |
| Poor | 56 to 65 | 32 to 37 |
| Extremely poor | > 66 | > 38 |

<Test Example 5: Suppression Test of Degradation Products of Composition Comprising PTN-SSS under High Temperature Condition>

In the stability test under high temperature conditions of Test Example 2, the types and amounts of degradation products were analyzed by HPLC. As a result, PTN-SS and PTN-SSSS were detected as degradation products of PTN-SSS, while a compound in which pantoic acid was eliminated by hydrolysis of the amide bond was not detected.

Figure 6 is a diagram showing a change over time in a value (%) obtained by expressing a peak area derived from PTN-SS generated under the same conditions as in Test Example 2 relative to a peak area derived from PTN-SSS. Figure 7 is a diagram showing a change over time in a value (%) obtained by expressing a peak area derived from PTN-SSSS generated under the same conditions as in Test Example 2 relative to a peak area derived from PTN-SSS. According to Figure 6 and Figure 7, in the aqueous solution of PTN-SSS, PTN-SS and PTN-SSSS were remarkably produced over time, whereas in the composition further comprising a saccharide or a silica gel-based carrier as a pharmaceutically acceptable carrier, remarkable production of these degradation products was not observed.

In PTN-SS, it is known that a compound in which pantoic acid is eliminated by hydrolysis of an amide bond is produced as a thermal decomposition product. On the other hand, in the thermal decomposition test of PTN-SSS of this Test Example, production of such a compound in which pantoic acid was eliminated was not detected. Then, unexpectedly, in the composition comprising PTN-SSS and a saccharide or a silica gel-based carrier as a pharmaceutically acceptable carrier, production of thermal degradation products (PTN-SS, PTN-SSSS) having completely different structures and production mechanisms from the thermal decomposition products in PTN-SS was remarkably suppressed. This result was further unexpected also from the viewpoint that production of thermal degradation products could be remarkably suppressed for PTN-SSS, which has lower stability than PTN-SS.

## Claims

1. A pharmaceutical composition comprising a trisulfide compound and a pharmaceutically acceptable carrier,

    wherein the trisulfide compound is at least one selected from the group consisting of:

    pantethine trisulfide or a pharmaceutically acceptable salt thereof;
    a compound represented by Formula (1):

    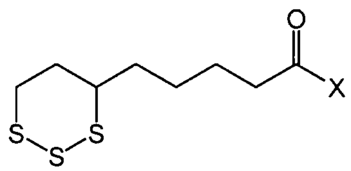

    (1)

    , wherein X represents $-OR^1$ or $-NR^2R^3$, $R^1$ represents a hydrogen atom or an alkyl group having 1 to 6 carbon atoms, $R^2$ and $R^3$ each independently represent a hydrogen atom or an alkyl group having 1 to 6 carbon atoms, and the alkyl group may have one or more substituents selected from the group consisting of an amino group and a carboxy group, or a pharmaceutically acceptable salt thereof; and
    glutathione trisulfide or a pharmaceutically acceptable salt thereof;

    and wherein the pharmaceutically acceptable carrier is at least one selected from the group consisting of a silica gel-based carrier and a saccharide.

2. The pharmaceutical composition according to claim 1, wherein X in the Formula (1) is -OH.

3. The pharmaceutical composition according to claim 1, wherein the trisulfide compound is pantethine trisulfide or a pharmaceutically acceptable salt thereof.

4. The pharmaceutical composition according to claim 1, wherein the pharmaceutically acceptable carrier is a silica gel-based carrier.

5. The pharmaceutical composition according to claim 1, wherein the trisulfide compound is pantethine trisulfide or a

pharmaceutically acceptable salt thereof, and the pharmaceutically acceptable carrier is a silica gel-based carrier.

6. The pharmaceutical composition according to any one of claims 1 to 5, wherein a mass ratio of the trisulfide compound to the pharmaceutically acceptable carrier in the pharmaceutical composition is 2:1 to 1:50.

7. The pharmaceutical composition according to any one of claims 1 to 5, wherein the pharmaceutical composition is for oral administration or transdermal administration.

8. The pharmaceutical composition according to claim 1, wherein the pharmaceutically acceptable carrier is a saccharide.

9. The pharmaceutical composition according to claim 1, wherein the pharmaceutically acceptable carrier is lactose.

10. The pharmaceutical composition according to claim 1, wherein the trisulfide compound is pantethine trisulfide or a pharmaceutically acceptable salt thereof, and the pharmaceutically acceptable carrier is lactose.

11. The pharmaceutical composition according to any one of claims 8 to 10, wherein a mass ratio of the trisulfide compound to the pharmaceutically acceptable carrier in the pharmaceutical composition is 1:1 to 1:20.

12. The pharmaceutical composition according to any one of claims 8 to 10, wherein the pharmaceutical composition is for oral administration, pulmonary administration, nasal administration, intraoral administration, rectal administration, or vaginal administration.

13. The pharmaceutical composition according to any one of claims 1 to 5 and 8 to 10, which is a carrier-adsorbed composition.

**Fig.1**

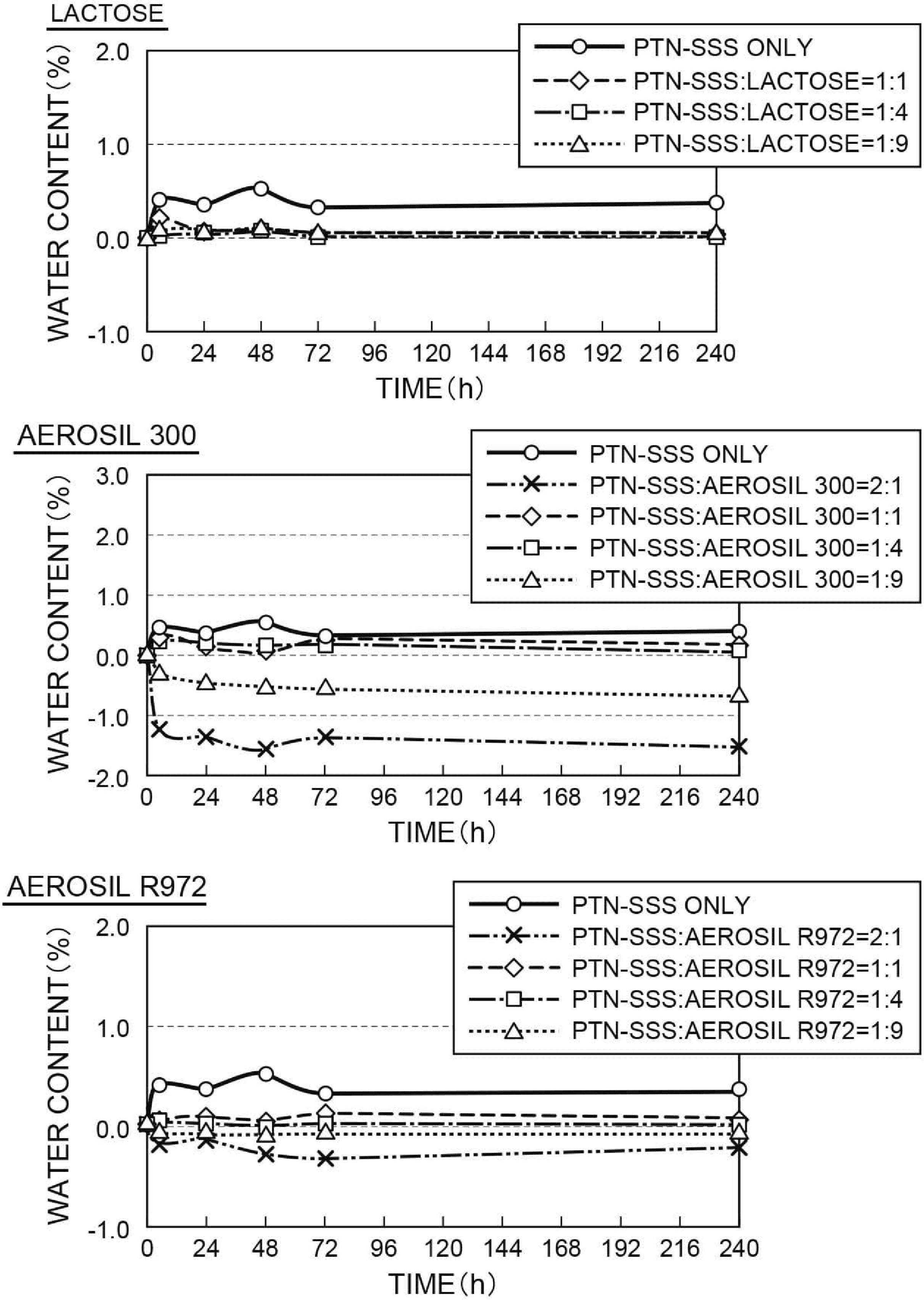
LACTOSE
WATER CONTENT (%)
2.0
1.0
0.0
-1.0
0
24
48
72
96
120
144
168
192
216
240
TIME (h)
PTN-SSS ONLY
PTN-SSS:LACTOSE=1:1
PTN-SSS:LACTOSE=1:4
PTN-SSS:LACTOSE=1:9
AEROSIL 300
WATER CONTENT (%)
3.0
2.0
1.0
0.0
-1.0
-2.0
TIME (h)
PTN-SSS ONLY
PTN-SSS:AEROSIL 300=2:1
PTN-SSS:AEROSIL 300=1:1
PTN-SSS:AEROSIL 300=1:4
PTN-SSS:AEROSIL 300=1:9
AEROSIL R972
WATER CONTENT (%)
2.0
1.0
0.0
-1.0
TIME (h)
PTN-SSS ONLY
PTN-SSS:AEROSIL R972=2:1
PTN-SSS:AEROSIL R972=1:1
PTN-SSS:AEROSIL R972=1:4
PTN-SSS:AEROSIL R972=1:9

Fig.2

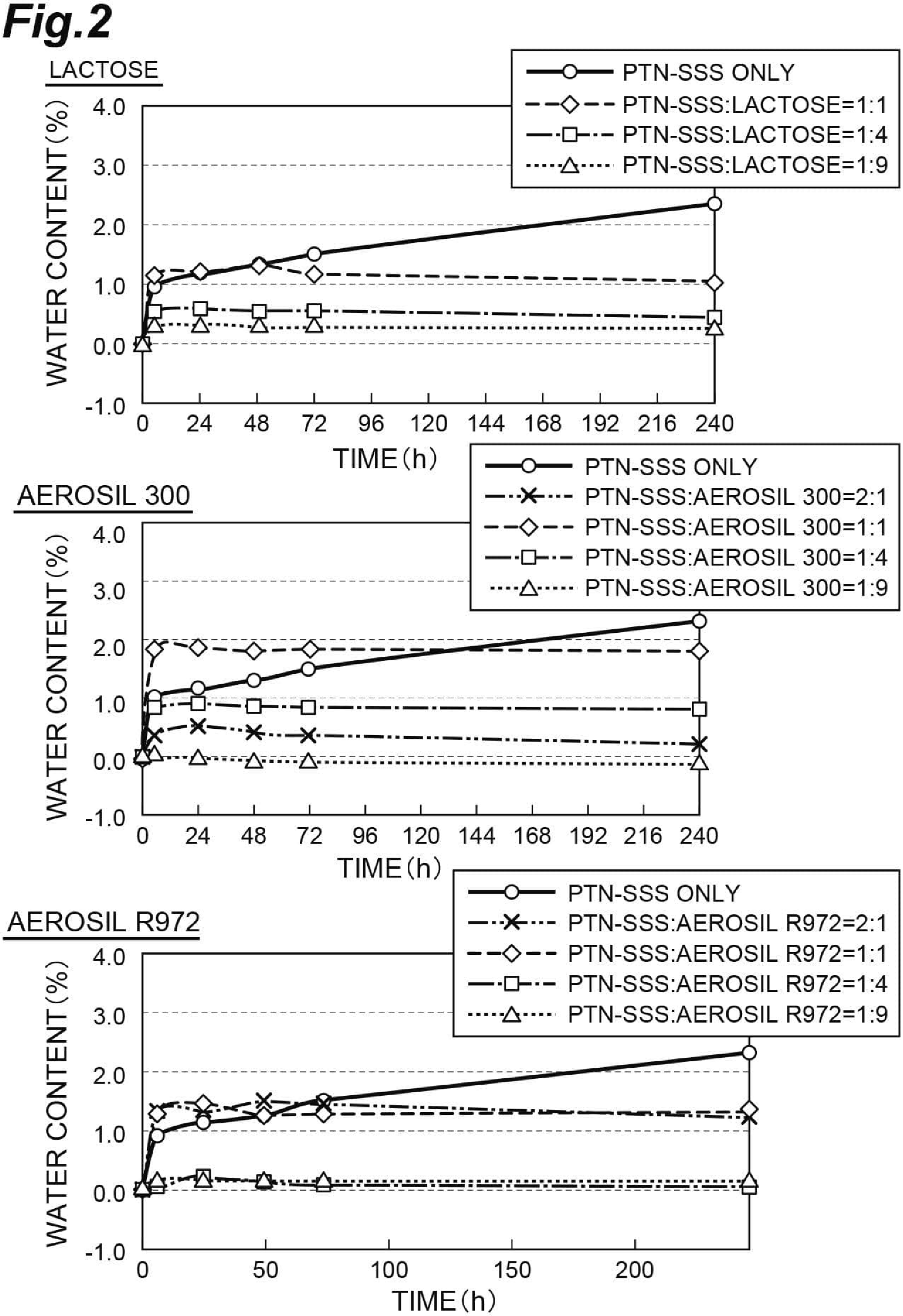
LACTOSE
PTN-SSS ONLY
PTN-SSS:LACTOSE=1:1
PTN-SSS:LACTOSE=1:4
PTN-SSS:LACTOSE=1:9
WATER CONTENT (%)
4.0
3.0
2.0
1.0
0.0
-1.0
0
24
48
72
96
120
144
168
192
216
240
TIME (h)
AEROSIL 300
PTN-SSS ONLY
PTN-SSS:AEROSIL 300=2:1
PTN-SSS:AEROSIL 300=1:1
PTN-SSS:AEROSIL 300=1:4
PTN-SSS:AEROSIL 300=1:9
WATER CONTENT (%)
4.0
3.0
2.0
1.0
0.0
-1.0
0
24
48
72
96
120
144
168
192
216
240
TIME (h)
AEROSIL R972
PTN-SSS ONLY
PTN-SSS:AEROSIL R972=2:1
PTN-SSS:AEROSIL R972=1:1
PTN-SSS:AEROSIL R972=1:4
PTN-SSS:AEROSIL R972=1:9
WATER CONTENT (%)
4.0
3.0
2.0
1.0
0.0
-1.0
0
50
100
150
200
TIME (h)

**Fig.3**

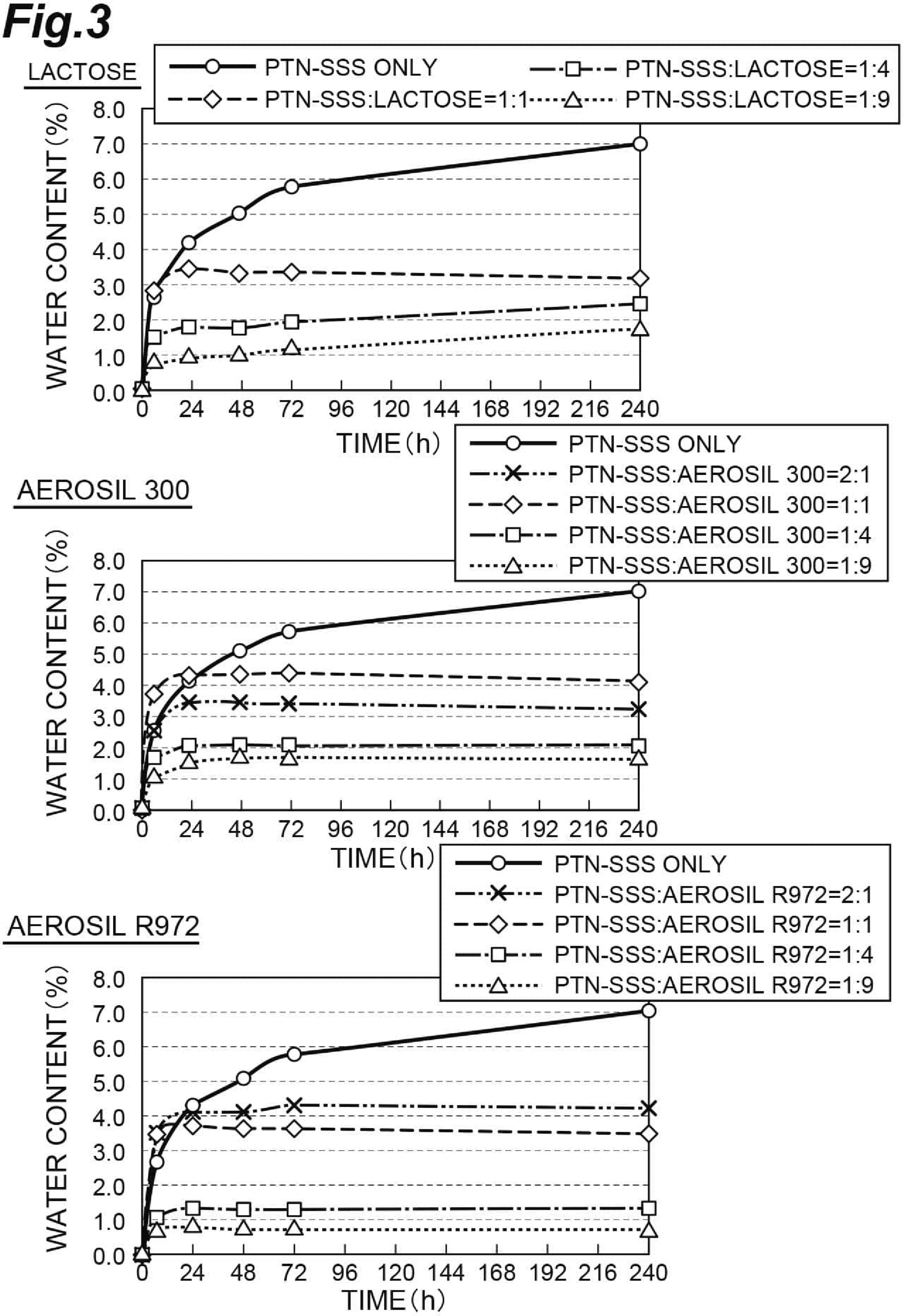

LACTOSE
PTN-SSS ONLY
PTN-SSS:LACTOSE=1:4
PTN-SSS:LACTOSE=1:1
PTN-SSS:LACTOSE=1:9
WATER CONTENT (%)
TIME (h)
AEROSIL 300
PTN-SSS ONLY
PTN-SSS:AEROSIL 300=2:1
PTN-SSS:AEROSIL 300=1:1
PTN-SSS:AEROSIL 300=1:4
PTN-SSS:AEROSIL 300=1:9
WATER CONTENT (%)
TIME (h)
AEROSIL R972
PTN-SSS ONLY
PTN-SSS:AEROSIL R972=2:1
PTN-SSS:AEROSIL R972=1:1
PTN-SSS:AEROSIL R972=1:4
PTN-SSS:AEROSIL R972=1:9
WATER CONTENT (%)
TIME (h)

# Fig.4

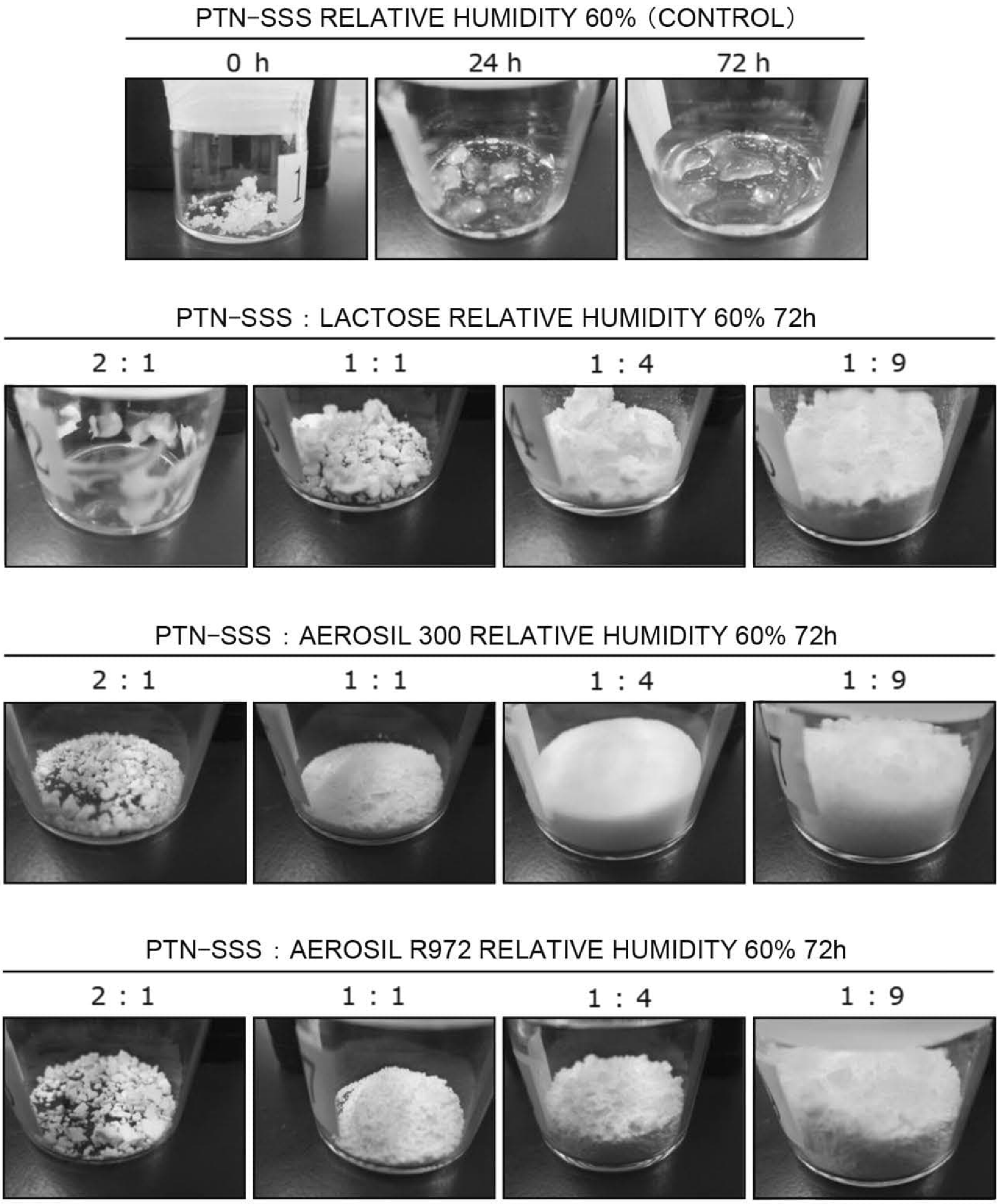
PTN-SSS RELATIVE HUMIDITY 60% (CONTROL)
0 h
24 h
72 h
1
PTN-SSS : LACTOSE RELATIVE HUMIDITY 60% 72h
2 : 1
1 : 1
1 : 4
1 : 9
PTN-SSS : AEROSIL 300 RELATIVE HUMIDITY 60% 72h
2 : 1
1 : 1
1 : 4
1 : 9
PTN-SSS : AEROSIL R972 RELATIVE HUMIDITY 60% 72h
2 : 1
1 : 1
1 : 4
1 : 9

*Fig.5*

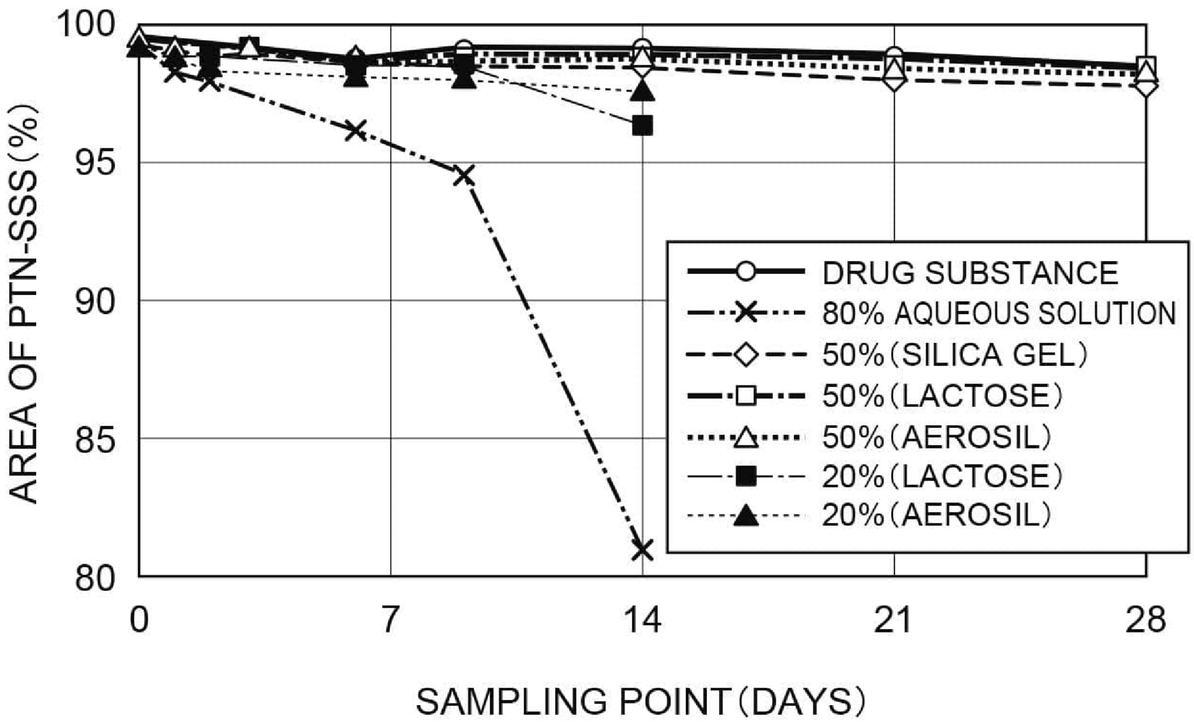
100
95
90
85
80
0
7
14
21
28
AREA OF PTN-SSS (%)
SAMPLING POINT (DAYS)
DRUG SUBSTANCE
80% AQUEOUS SOLUTION
50% (SILICA GEL)
50% (LACTOSE)
50% (AEROSIL)
20% (LACTOSE)
20% (AEROSIL)

***Fig.6***

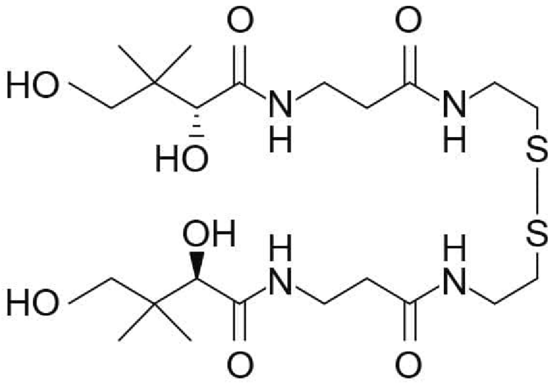

PTN-SS

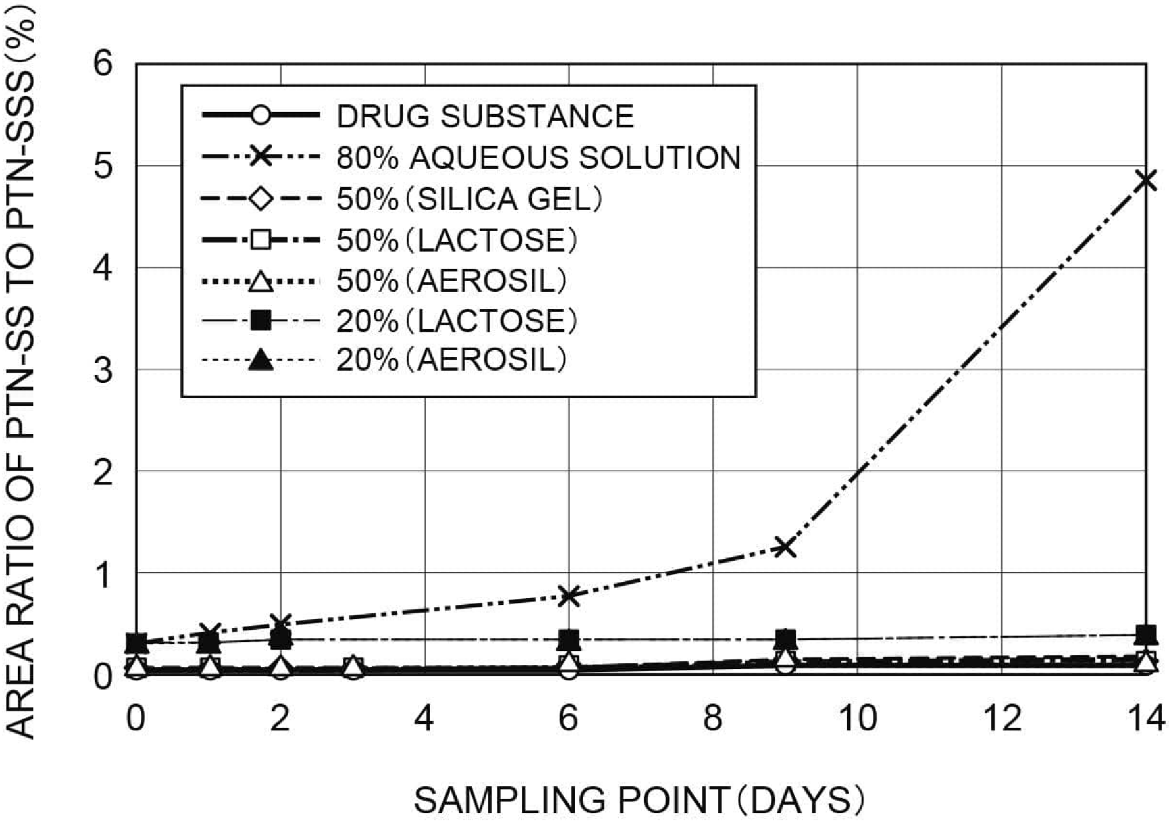
AREA RATIO OF PTN-SS TO PTN-SSS (%)
0
1
2
3
4
5
6
DRUG SUBSTANCE
80% AQUEOUS SOLUTION
50% (SILICA GEL)
50% (LACTOSE)
50% (AEROSIL)
20% (LACTOSE)
20% (AEROSIL)
0
2
4
6
8
10
12
14
SAMPLING POINT (DAYS)

# Fig.7

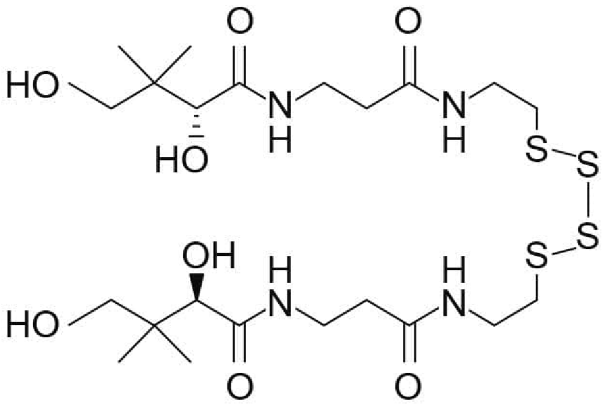

PTN-SSSS

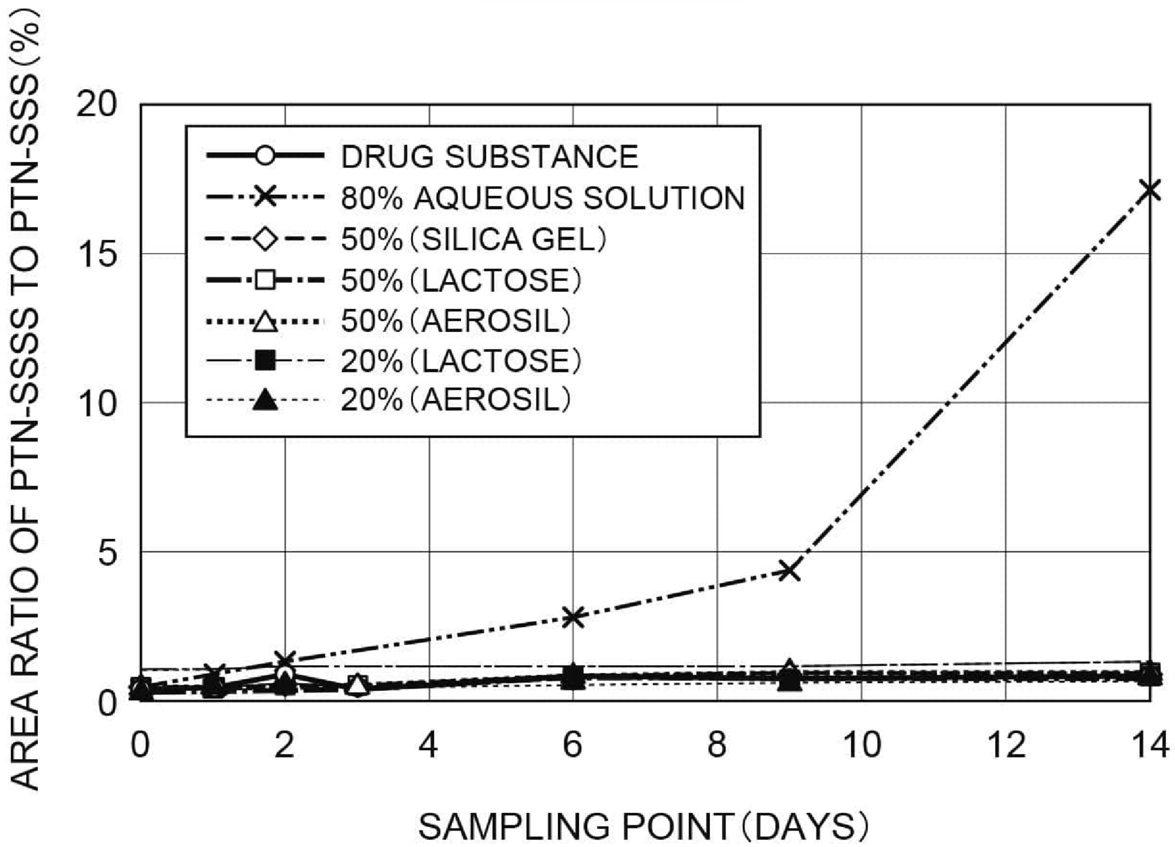

## INTERNATIONAL SEARCH REPORT

International application No.
**PCT/JP2024/034101**

**A. CLASSIFICATION OF SUBJECT MATTER**

***A61K 31/198***(2006.01)i; ***A61K 31/385***(2006.01)i; ***A61K 47/04***(2006.01)i; ***A61K 47/26***(2006.01)i
FI: A61K31/198; A61K31/385; A61K47/04; A61K47/26

According to International Patent Classification (IPC) or to both national classification and IPC

**B. FIELDS SEARCHED**

Minimum documentation searched (classification system followed by classification symbols)

A61K31/198; A61K31/385; A61K47/04; A61K47/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/158894 A1 (KYOUWA HAKKO BIO CO., LTD.) 06 August 2020 (2020-08-06) claims, paragraphs [0014]-[0017], [0019], [0028], examples 1, 3-4 | 1-2, 6-8, 12-13 |
| Y | claims, paragraphs [0014]-[0017], [0019], [0028], examples 1, 3-4 | 1-3, 6-9, 11-13 |
| A | claims, paragraphs [0014]-[0017], [0019], [0028], examples 1, 3-4 | 4-5, 10 |
| Y | WO 2022/045052 A1 (KYOWA PHARMA CHEMICAL CO., LTD.) 03 March 2022 (2022-03-03) claims, examples | 1-3, 6-9, 11-13 |
| A | claims, examples | 4-5, 10 |
| X | WO 2023/282269 A1 (KYOWA PHARMA CHEMICAL CO., LTD.) 12 January 2023 (2023-01-12) claims, paragraphs [0020]-[0051], reference examples 3-9 | 1-2, 6-8, 11-13 |
| A | claims, paragraphs [0020]-[0051], reference examples 3-9 | 3-5, 9-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/034101

C. DOCUMENTS CONSIDERED TO BE RELEVANT

| Category* | Citation of document, with indication, where appropriate, of the relevant passages | Relevant to claim No. |
|---|---|---|
| A | HAMID. Hisyam Abdul et al. Polysulfide stabilization by tyrosine and hydroxyphenyl-containing derivatives that is important for a reactive sulfur metabolomics analysis. Redox Biology. 02 January 2019, vol. 21, p. 10109, p. 10109, DOI: 10.1016/j.redox.2019.101096<br>entire text, in particular, abstract, fig. 3F | 1-13 |
| A | WO 2018/117186 A1 (KYOUWA HAKKO BIO CO., LTD.) 28 June 2018 (2018-06-28)<br>entire text, in particular, example 4 | 1-13 |

Form PCT/ISA/210 (second sheet) (July 2022)

**INTERNATIONAL SEARCH REPORT**
**Information on patent family members**

International application No.
**PCT/JP2024/034101**

| Patent document cited in search report | Publication date (day/month/year) | Patent family member(s) | Publication date (day/month/year) |
|---|---|---|---|
| WO 2020/158894 A1 | 06 August 2020 | JP 2022-74166 A<br>claims, paragraphs [0014]-[0017], [0019], [0082], examples 1, 3-4 | |
| WO 2022/045052 A1 | 03 March 2022 | US 2023/0357141 A1<br>claims, examples<br>EP 4194437 A1<br>claims, examples<br>CN 115943140 A<br>claims, examples | |
| WO 2023/282269 A1 | 12 January 2023 | EP 4342464 A1<br>claims, reference examples<br>CN 117320709 A<br>claims, reference examples | |
| WO 2018/117186 A1 | 28 June 2018 | US 2020/0079818 A1<br>entire text, in particular, example 4<br>EP 3560947 A1<br>entire text, in particular, example 4<br>CN 110088118 A<br>entire text, in particular, example 4 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022045052 A
- WO 2022045212 A
- WO 2021200487 A


### Non-patent literature cited in the description


- **FUMIAKI NAGASHIMA et al.** Sulfide:quinone oxidoreductase ameliorates neurodegeneration in a murine model of Parkinson's disease. *Redox Biology*, 2023, vol. 59, 102562
- General Information, Powder Flow. The Japanese Pharmacopoeia